# EUROPÄISCHE PATENTANMELDUNG

(11) **EP 2 226 122 A1**
(43) Veröffentlichungstag der Anmeldung: **08.09.2010**
(21) Anmeldenummer: 09002120.5
(22) Anmeldetag: 16.02.2009
(51) Int. Cl.: B01L 3/00, B65D 17/00, A61B 5/00, A45D 44/00, A45D 42/00

(54) **Behälter mit Referenzlösung für die klinische Chemie**

(71) Anmelder: Roche Diagnostics GmbH, 68305 Mannheim (DE); F.HOFFMANN-LA ROCHE AG, 4070 Basel (CH)
(72) Erfinder: Sternberger, Christa, 68766 Hockenheim (DE); Ahl, Axel, 68167 Mannheim (DE)
(74) Vertreter: Twelmeier Mommer & Partner

(57) **Zusammenfassung**

Die Erfindung betrifft einen Behälter mit darin enthaltener Referenzlösung (6) für die klinische Chemie, insbesondere Glucoselösung für ein Messsystem zur Bestimmung der Glucosekonzentration einer Körperflüssigkeitsprobe. Erfindungsgemäß ist vorgesehen dass der Behälter einen Hals (2) mit einer Sollbruchstelle (3) aufweist und in dem Behälter ein Applikator (5) zum Applizieren von Glucoselösung (6) angeordnet ist.

## Beschreibung

Die Erfindung betrifft einen Behälter mit darin enthaltener Referenzlösung für die klinische Chemie, beispielsweise Glucoselösung, Lactatlösung, Cholesterinlösung oder Hämoglobinlösung.

Glucoselösung wird zum Testen oder Kalibrieren von Messgeräten benötigt, die von Diabetikern zur Bestimmung ihres Blutzuckerspiegels verwendet werden. Hersteller von Messsystemen zur Bestimmung der Glucosekonzentration von Körperflüssigkeitsproben bieten deshalb als Teil solcher Messsysteme Behälter mit Glucoselösung einer definierten Konzentration, typischerweise im Bereich von 50 bis 300 mg/dl, an. Zum Testen oder Kalibrieren eines Messgeräts kann derartige Glucoselösung beispielsweise auf einen Teststreifen aufgebracht und anschließend die Glucosekonzentration gemessen werden. Da die Konzentration der Lösung bekannt ist, kann auf diese Weise ein Messgerät kontrolliert oder kalibriert werden.

Für unter der Marke ACCU-CHEK vertriebe Systeme wird Glucoselösung in Tropfflaschen angeboten, die für mehrer Applikationen 4 ml Lösung enthalten. Zum Applizieren von Glucoselösung wird ein Flaschendeckel abgeschraubt und ein Tropfen auf einen Teststreifen aufgebracht. Die Öffnung der Flaschen hat dabei eine bei Medikamentenfläschchen übliche Form, die das Erzeugen einzelner Tropfen erleichtert, indem man die Flasche mit der Öffnung senkrecht nach unten hält.

Für Messsysteme zur Messung anderer Analyten in Körperflüssigkeitsproben, beispielsweise Lactat oder Cholesterin, werden entsprechen andere Analytlösung benötigt, also beispielsweise Lactat- oder Cholesterinlösung.

Aufgabe der Erfindung ist es, einen Weg aufzuzeigen, wie insbesondere Menschen, deren Geschicklichkeit durch Alter oder Krankheit eingeschränkt ist, das Applizieren einer Referenzlösung, insbesondere von Glucoselösung, erleichtert und zugleich die Gefahr einer Beeinträchtigung der Referenzlösung möglichst zuverlässig ausgeschlossen werden kann.

Diese Aufgabe wird durch einen Behälter mit den im Anspruch 1 angegebenen Merkmalen gelöst. Vorteilhafte Weiterbildungen der Erfindung sind Gegenstand von Unteransprüchen.

Durch die erfindungsgemäße Maßnahme, dass der Behälter einen Hals mit einer Sollbruchstelle aufweist und in dem Behälter ein Applikator zum Applizieren von Glucoselösung angeordnet ist, lässt sich die Handhabung wesentlich vereinfachen. Insbesondere können mit einem Applikator auch kleinere Mengen von beispielsweise weniger als 5 µl, insbesondere also kleinere Mengen als ein Tropfen, sehr präzise auf ein Testfeld aufgetragen werden. Mit einem Applikator hat ein Benutzer das Aufbringen von Glucoselösung voll unter Kontrolle, was gegenüber herkömmlichen Flaschen ein wichtiger Vorteil ist, da sich dort der Zeitpunkt, zu dem sich ein Tropfen löst, nicht vorherbestimmen lässt und deshalb die Notwendigkeit besteht, das Fläschchen über längere Zeit ruhig zu halten. Bei Verwendung eines Applikators kann der Benutzer den Zeitpunkt der Applikation dagegen selbst vorgeben und diese rasch durchführen. Zudem lässt sich ein Behälter, der einen Hals mit einer Sollbruchstelle aufweist, vorteilhaft leicht öffnen und nicht wieder verschließen. Da folglich bei jeder Applikation ein frischer Behälter geöffnet werden muss, ist gewährleistet, dass die Referenzlösung in einwandfreiem Zustand ist, also die erwartete Konzentration hat. Insbesondere ist die Lösung durch Verwendung eines erfindungsgemäßen Behälters vor Verkeimung geschützt. Dies ist insbesondere für Glucoselösung bedeutsam, da Keime die Glucosekonzentration herabsetzen können.

Die Sollbruchstelle kann beispielsweise eine Schwächung in einer Behälterwand sein. Bevorzugt ist die Sollbruchstelle eine Nut, insbesondere eine Ringnut. Die Behälterwand mit der Sollbruchstelle ist bevorzugt durch Spritzgießen aus Kunststoff hergestellt.

Eine vorteilhafte Weiterbildung der Erfindung sieht vor, dass der Applikator an dem Behälter befestigt ist. Auf diese Weise kann der Applikator nach dem Öffnen des Behälters mittels des ihn tragenden Behälterteils leicht gehandhabt werden. Prinzipiell ist es jedoch auch möglich, dass der Applikator lose in dem Behälter angeordnet ist.

Weitere Einzelheiten und Vorteile der Erfindung werden an einem Ausführungsbeispiel unter Bezugnahme auf die beigefügten Abbildungen erläutert. Es zeigen:
- Figur 1:: ein Ausführungsbeispiel eines erfindungsgemäßen Behälters;
- Figur 2:: der in Figur 1 gezeigte Behälter nach Entfernen des Verschlussteils;
- Figur 3:: eine Schnittansicht zu Figur 1; und
- Figur 4:: ein Ausführungsbeispiel eines Messgeräts zur Bestimmung der Glucosekonzentration.

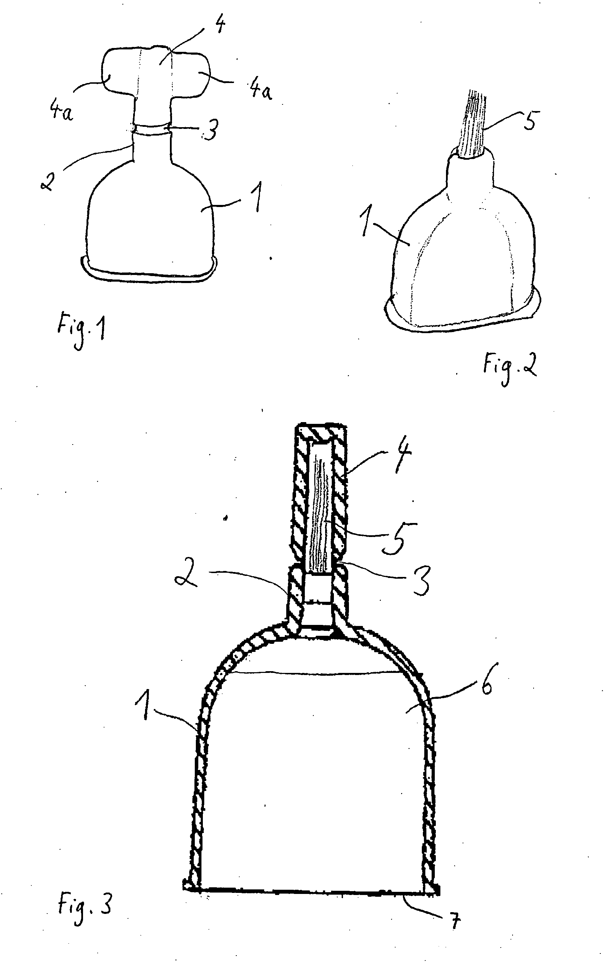

Der in den Figuren 1 bis 3 dargestellte Behälter hat einen als Flasche ausgebildeten Hauptteil 1, der über eine an einem Hals 2 Sollbruchstelle 3 mit einem Verschlussteil 4 verbunden ist. Die Sollbruchstelle 3 ist beispielsweise als eine Ringnut in dem Hals 2 ausgebildet. Das Verschlussteil 4 lässt sich durch Abdrehen von dem Hauptteil 1 trennen und hat zu diesem Zweck zwei Flügel 4a, über die sich ein Drehmoment übertragen lässt. Die Sollbruchstelle 3 kann so auf Torsion beansprucht werden, was schließlich zum Bruch führt. Alternativ kann der Behälter aber auch so ausgebildet werden, dass die Sollbruchstelle 3 durch Biegebeanspruchung zum Brechen gebracht oder aufgerissen wird.

In dem Behälter ist ein mit Glucoselösung 6 getränkter Applikator 5 angeordnet und an dem Behälter befestigt. Der Applikator 5 ist bevorzugt an dem Hauptteil 1 befestigt, kann prinzipiell aber auch an dem Verschlussteil 4 befestigt sein. Bei dem dargestellten Ausführungsbeispiel sitzt der Applikator 5 in dem Hals 2 und ragt in das hohl ausgebildete Verschlussteil 4 hinein. Der Applikator 5 verschließt den Hals 2 flüssigkeitsdurchlässig.

Nach dem Entfernen des Verschlussteils 4 kann deshalb in dem Behälter enthaltene Glucoselösung 6 durch den Applikator 5 hindurch austreten und beispielsweise auf einem Testfeld appliziert werden. Der Applikator 5 kann beispielsweise einen Schwamm, Docht oder Flies aufweisen und ähnlich wie die Spitze eines Faserschreibers zum Applizieren der in dem Behälter enthaltenen Flüssigkeit verwendet werden. Bei dem dargestellten Ausführungsbeispiel weist der Applikator 5 jedoch Fasern auf, die einen Pinsel bilden. Der Pinsel ist als ein Durchflusspinsel ausgebildet, so dass die Glucoselösung 6 aus dem Hauptteil 1 des Behälters durch den Hals 2 hindurch in den Pinsel strömen kann.

Ein geeigneter Pinsel kann vorteilhaft hergestellt werden, indem ein Bündel von Kunststofffasern an einem Ende verbunden wird, beispielsweise verklebt oder verschweißt wird, wobei ein oder mehrere Dorne durch den Verbindungsbereich hindurchragen, um einen oder mehrere Durchtrittskanäle freizuhalten. Nach dem Verkleben oder Verschweißen wird der Dorn bzw. die Dorne entfernt, so dass Flüssigkeitskanäle verbleiben. Der so gebildete Pinsel kann dann im Hals 2 des Behälters befestigt werden, beispielsweise durch Einpressen, Kleben oder Schweißen.

Eine umlaufende Behälterwand des Hauptteils 1 und das Verschlussteil 4 sind bevorzugt einstückig als Spritzgussteil ausgebildet. Bei dem dargestellten Ausführungsbeispiel wird der Boden des Hauptteils 1 von einer Folie 7 gebildet. Auf diese Weise kann der Boden des Behälters als eine vorteilhaft große Einfüllöffnung zum Einbringen des Applikators 5 und anschließend zum Einfüllen von Glucoselösung 6 genutzt werden. Wenn die Einfüllöffnung nicht mehr gebraucht wird, kann sie mit einer Folie 7 dicht verschlossen werden.

Der Behälter kann nach dem Einbringen von Glucoselösung 6 beispielsweise auch dadurch verschlossen werden, dass er an dem dortigen Ende zusammengepresst und die dabei aneinander angelegten Ränder miteinander verklebt oder verschweißt werden. Auf diese Weise werden beispielsweise Tuben nach dem Füllen verschlossen.

Die als Boden des Hauptteils 1 verwendete Folie 7 ist bevorzugt eine Verbundfolie mit einer Metallschicht und einer Kunststoffschicht. Besonders bevorzugt ist die Folie 7 eine Aluminiumverbundfolie. Die Aluminiumverbundfolie hat neben einer Aluminiumschicht mindestens eine Kunststoffschicht, bevorzugt zwei Kunststoffschichten, zwischen denen die Aluminiumschicht angeordnet ist. Die Kunststoffschichten können beispielsweise aus Polyethylen und/oder Polypropylen bestehen. Die Kunststoffschichten können auflaminierte Folien sein oder beispielsweise als Lack aufgetragen sein.

Die Folie 7 kann mit der umlaufenden Behälterwand des sich zu dem Hals 2 hin verjüngenden Hauptteils 1 verschweißt werden. Hierfür ist es günstig, die Behälterwand und die Folie 7 bzw. die Innenseite einer Verbundfolie aus demselben Kunststoff zu bilden, beispielsweise aus Polypropylen.

Die in dem fertigen Behälter enthaltene Glucoselösung 6 kann beispielsweise eine Konzentration zwischen 40 mg/dl und 150 mg/dt haben. Bei dem dargestellten Ausführungsbeispiel wird Glucoselösung 6 mit einer Konzentration von 50 mg/dl verwendet. In dem Behälter sind beispielsweise 0,5 ml bis 1,5 ml Glucoselösung enthalten.

Die Konzentration der Glucoselösung 6 ist auf dem Behälter angegeben, beispielsweise auf einem Etikett auf der Seitenwand. Chargeninformationen, beispielsweise das Herstellungsdatum oder ein Mindesthaltbarkeitsdatum, können auf die den Boden bildende Folie 7 aufgedruckt werden.

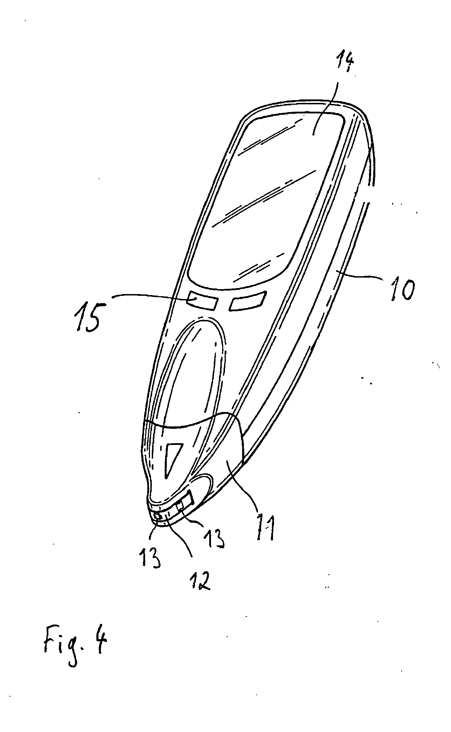

Der beschriebene Behälter mit Glucoselösung 6 ist besonders vorteilhaft für ein Messsystem geeignet, das als weitere Teile ein in Figur 4 gezeigtes Messgerät 10 und eine Bandkassette 11 aufweist, die ein Trägerband 12 enthält, das Testfelder 13 mit Nachweisreagenzien trägt. Beim Aufbringen von Glucoselösung 6 auf ein Testfeld 13 eines Trägerbandes 12 ist es nämlich besonders wichtig, dass nicht zu viel Glucoselösung 6 aufgetragen wird, da dies zu einer Verschmutzung des gesamten Trägerbandes 12 führen kann.

Das in Figur 4 gezeigte Messgerät 10 hat Bedienungselemente 15, beispielsweise Tasten, und ein Fach, in das eine Bandkassette 11 eingesetzt werden kann. Messergebnisse können mit einer Anzeigeeinrichtung 14, beispielsweise einem Flüssigkristalldisplay, anzeigt werden.

### Bezugszeichen

- 1: Hauptteil
- 2: Hals
- 3: Sollbruchstelle
- 4: Verschlussteil
- 4a: Flügel
- 5: Applikator
- 6: Glucoselösung
- 7: Folie
- 10: Messgerät
- 11: Bandkassette
- 12: Trägerband
- 13: Testfeld
- 14: Anzeigeeinrichtung
- 15: Bedienungselement

## Patentansprüche

1. Behälter mit darin enthaltener Referenzlösung (6) für die klinische Chemie, insbesondere für ein Messsystem zur Bestimmung der Glucosekonzentration einer Körperflüssigkeitsprobe, **dadurch gekennzeichnet, dass** der Behälter einen Hals (2) mit einer Sollbruchstelle (3) aufweist und in dem Behälter ein Applikator (5) zum Applizieren von Glucoselösung (6) angeordnet ist.

2. Behälter nach Anspruch 1, **dadurch gekennzeichnet, dass** der Applikator (5) mit Referenzlösung (6) getränkt ist.

3. Behälter nach einem vorstehenden Ansprüche, **dadurch gekennzeichnet, dass** der Applikator (5) Fasern aufweist.

4. Behälter nach einem vorstehenden Ansprüche, **dadurch gekennzeichnet, dass** der Applikator (5) ein Pinsel ist.

5. Behälter nach einem der vorstehenden Ansprüche, **dadurch gekennzeichnet, dass** der Applikator (5) an dem Behälter befestigt ist.

6. Behälter nach einem der vorstehenden Ansprüche, **dadurch gekennzeichnet, dass** der Behälter einen hohlen Hauptteil (1) aufweist, der über die Sollbruchstelle (3) mit einem Verschlussteil (4) verbunden ist.

7. Behälter nach Anspruch 6, **dadurch gekennzeichnet, dass** der Applikator (5) an dem Hauptteil (1) befestigt ist.

8. Behälter nach Anspruch 7, **dadurch gekennzeichnet, dass** der Applikator (5) in dem Hals (2) sitzt und diesen flüssigkeitsdurchlässig verschließt.

9. Behälter nach einem der Ansprüche 6 bis 8, **dadurch gekennzeichnet, dass** das Verschlussteil (4) hohl ist.

10. Behälter nach einem der Ansprüche 6 bis 9, **dadurch gekennzeichnet, dass** das Verschlussteil (4) abdrehbar ist.

11. Behälter nach einem der Ansprüche 6 bis 10, **dadurch gekennzeichnet, dass** das Verschlussteil (4) zwei Flügel (4a) zum Abdrehen des Verschlussteils (4) aufweist.

12. Behälter nach einem der vorstehenden Ansprüche, **dadurch gekennzeichnet, dass** die Sollbruchstelle (3) eine Nut ist.

13. Behälter nach einem der vorstehenden Ansprüche, **dadurch gekennzeichnet, dass** der Hauptteil (1) einen Boden aus Folie (7) hat.

14. Behälter nach einem der vorstehenden Ansprüche, **dadurch gekennzeichnet, dass** eine umlaufende Behälterwand des Hauptteils (1) und das Verschlussteil (4) einstückig als Spritzgussteil ausgebildet sind.

15. System zum Messen einer Analytkonzentration, insbesondere der Glucosekonzentration, einer menschlichen oder tierischen Körperflüssigkeitsprobe, mit
einem Messgerät (10),
einer Bandkassette (11), die ein Trägerband (12) enthält, das Testfelder (13) mit Nachweisreagenzien trägt, und
einem Behälter (1, 4) nach einem der vorstehenden Ansprüche.
